# EUROPEAN PATENT SPECIFICATION

(11) **EP 2 883 050 B1**
(45) Date of publication and mention of the grant of the patent: **31.08.2016**
(21) Application number: 13759853.8
(22) Date of filing: 23.07.2013
(51) Int. Cl.: G01N 33/543, G01N 33/558

(54) **MEMBRANE ASSEMBLY AND A LATERAL FLOW IMMUNOASSAY DEVICE COMPRISING SUCH MEMBRANE ASSEMBLY**
MEMBRANANORDNUNG UND SEITLICHE FLUSSIMMUNOASSAY-VORRICHTUNG MIT EINER SOLCHEN MEMBRANANORDNUNG
ENSEMBLE DE MEMBRANES ET DISPOSITIF POUR DOSAGE IMMUNOLOGIQUE À ÉCOULEMENT LATÉRAL COMPRENANT UN TEL ENSEMBLE DE MEMBRANES

(30) Priority: 09.08.2012 EP 12179818
(43) Date of publication of application: 17.06.2015
(73) Proprietor: Stichting Dienst Landbouwkundig Onderzoek, 6708 PB Wageningen (NL)
(72) Inventor: VAN AMERONGEN, Aart, NL-3906 ES Veenendaal (NL); WICHERS, Jan Herman, NL-6715 LS Ede (NL)
(74) Representative: Nederlandsch Octrooibureau
(86) International application number: PCT/NL2013/050548
(87) International publication number: WO 2014/025251

(56) References cited:
- EP-A2- 0 553 770
- US-A- 5 252 496
- US-A1- 2006 019 406

## Description

### Field of the Invention

The invention relates to an elongate membrane assembly comprising a microporous membrane layer supported on a liquid-impermeable support layer and to a lateral flow immunoassay device comprising such membrane assembly.

### Background of the Invention

Immunoassays are often used to detect the presence or the concentration of various substances, often referred to as ligands, in biological fluids such as blood, urine or saliva. In a solid phase immunoassay, a receptor, typically an antibody which is specific for the ligand to be detected, is immobilised on a solid support. A test fluid that may comprise the ligand to be detected is contacted with the solid support and a receptor-ligand pair is formed in case the ligand is present. In order to make the receptor-ligand pair visible, labeled antibodies may be used that bind to the receptor-ligand pair followed by visual detection of the labeled antibody bound to the receptor-ligand pair.

In so-called sandwich immunoassays, a ligand is sandwiched between a labeled antibody and an antibody immobilised on a solid support. Such assay is for example described in US 5,591,645.

Porous materials such as nitrocellulose, nylon, cellulose acetate, glass fibres and other porous polymers have been employed as solid supports in solid phase immunoassays. In so-called lateral flow assays, a fluid wherein a ligand is to be detected is applied to one end of a porous membrane layer and flows in lateral direction through the membrane under the action of capillary forces. Such membrane comprises immobilised receptor that is capable of binding the ligand to be detected. The immobilised receptor may be evenly distributed over the entire membrane. Typically, however, such immobilised receptor is located in defined test or detection zones in the membrane, usually in narrow test lines that have been applied by means of inkjet printing or other aerosol spraying techniques.

In lateral flow immunoassays, typically a thin layer of microporous material with immobilised receptor is supported on a liquid-impermeable layer to provide sufficient rigidity to the fragile membrane layer. Usually a layer of microporous material with a thickness in the range of from 100 to 200 µm is supported on a support layer, usually referred to as 'membrane backing'.

A general issue with lateral flow immunoassays is assay sensitivity and therewith signal intensity. Such assays are typically five to ten times less sensitive than for example an Enzyme-Linked Immuno Sorbent Assay (ELISA). Several measures to improve the signal have been proposed, for example signal amplification strategies such as enzymatic enhancement of the signal, but there is still room for improvement.

### Summary of the Invention

It has now been found that immobilisation of receptor by spraying techniques such as inkjet printing or other aerosol spraying techniques on a microporous membrane such as a nitrocellulose membrane, typically results in the receptor being immobilised in only a thin upper layer of the membrane. Confocal laser scanning microscopy analysis of nitrocellulose membranes on which antibodies were immobilised by means of a nitrogen-driven spraying technique showed that the antibodies are typically immobilised in the upper part of the membrane layer. Usually in the upper 80 µm of the membrane layer, or even only in the upper 60 µm or upper 30 µm of the membrane layer. Since microporous membranes in lateral flow assays are usually much thicker than 60 or 80 µm, often in the range of from 100 to 200 µm, usually of from 120 to 180 µm, only a small part of the test fluid will be contacted with immobilised receptor.

Use of a lateral flow membrane with a thickness that is not substantially larger than the thickness of the layer in which antibodies are immobilised in immunoassays, will importantly improve the sensitivity of lateral flow immunoassays.

Moreover, it has been found that, even if a receptor penetrates deeper into the membrane and is also immobilised in lower parts of the membrane, many labeling techniques such as for example those using specifically-bound coloured or fluorescent nanoparticles, will only allow a detectable signal from nanoparticles bound at or just below the membrane surface. Specifically-bound nanoparticles in lower parts of the membrane will not contribute to the signal, which is usually recorded from above the membrane. Therefore, in those cases the sensitivity of the assay will be substantially increased if nanoparticles that have bound the analyte to be detected will be captured (sandwich-type assays) or not captured (inhibition-type assays) at or near the surface of the membrane.

Accordingly, the present invention relates to an elongate membrane assembly having a length, a width and a height, the assembly comprising a microporous membrane layer supported on a liquid-impermeable support layer and the assembly being suitable for lateral flow of a liquid through the membrane layer under the action of capillary forces, wherein the assembly has a constant height and at least part of the membrane layer has a first thickness in the range of from 20 to 80 µm over the entire width of the assembly.

The membrane assembly can advantageously be applied in a solid phase lateral flow immunoassay. The membrane assembly then further comprises a ligand-binding molecule immobilised in the membrane layer, preferably in one or more detection zones. In such solid phase lateral flow immunoassay, the membrane layer has at least in the one or more detection zones the first thickness, i.e. a thickness in the range of from 20 to 80 µm.

An important advantage of having at least in the detection zones a membrane layer with a thickness that is not substantially larger than the depth of the layer in which ligand-binding molecules are immobilized and/or in which detection signals will be recorded, is that in the detection zones, the liquid to be analysed is forced to flow through the part of the membrane layer that comprises immobilised ligand-binding molecules and/or allows detection signals to be recorded. This will result in an increased number of ligands having interaction with immobilised ligand-binding molecules and/or an increased number of signal entities available for recording. Therewith, the sensitivity of the immunoassay in terms of signal intensity is increased.

In one embodiment of the invention, the membrane layer has a constant thickness, i.e. having a thickness in the range of from 20 to 80 µm over its entire length and width. Preferably, however, the membrane layer has the first thickness in the detection zones and a second, larger thickness outside the detection zones. Having a larger thickness outside the detection zones has several advantages, including a less fragile membrane layer resulting in improved handability and an increased capacity for test liquid.

In a further aspect, the invention relates to a lateral flow immunoassay device comprising the membrane assembly as hereinbefore defined.

### Summary of the Drawings

In Figure 1 is shown a longitudinal section of a membrane assembly according to the invention.
In Figure 2 is shown a longitudinal section of a lateral flow immunoassay device according to the invention.
In Figure 3 is shown the results of confocal laser scanning microscopy experiments showing the depth of antibody immobilisation in different nitrocellulose membranes.

### Detailed Description of the Invention

The membrane assembly according to the invention is an elongate assembly of a microporous membrane layer supported on a liquid-impermeable support layer. The assembly is suitable for lateral flow of a liquid through the membrane layer under the action of capillary forces and is typically used in lateral flow immunoassays for detecting a ligand or analyte in a test fluid that flows laterally through the microporous membrane layer. The elongate assembly has a length, a width and a height. The height of the assembly, i.e. support layer plus membrane layer, is constant. At least part of the membrane layer has a first thickness in the range of from 20 to 80 µm, preferably of from 20 to 60 µm, more preferably of from 20 to 40 µm, even more preferably of from 20 to 30 µm over the entire width of the assembly.

In the assembly according to the invention, the entire membrane layer may have the first thickness. Thus the assembly comprises a support layer and a membrane layer that each have a constant thickness. Preferably, however, the membrane layer in the assembly has, in lateral direction, alternating the first thickness and a second, larger thickness, each over the entire width of the assembly. The second thickness is preferably the thickness that microporous membrane layers in known lateral flow immunoassays have. More preferably the second thickness is in the range of from 100 to 200 µm, even more preferably of from 120 to 180 µm. Since the assembly of membrane and support layer has a constant height, the support layer will decrease in thickness where the membrane layer increases in thickness and *vice versa*.

If used in a lateral flow immunoassay device, the membrane assembly according to the invention is preferably such that the membrane layer has the first thickness in zones wherein ligand-binding molecule is immobilised in the membrane layer (detection zones) and, if present, also in zones of the membrane layer comprising a moveable conjugate of ligand-binding molecule and marker (conjugate zones) and has the second thickness upstream and downstream of such zones and between such zones.

The thickness of the membrane layer may decrease and increase in any suitable way, for example step-wise or gradually. Preferably, the thickness of the membrane layer is gradually decreasing from second to first thickness. Reference herein to gradually decreasing is to a decrease with a gradient and not step-wise, i.e. a decrease with a finite tangent (decrease in thickness per unit of length of membrane). More preferably, the thickness is decreasing with at most 300% (3 µm decrease in thickness over 1 µm membrane length), even more preferably with at most 200%, even more preferably the decrease is in the range of from 30 to 100%.

The membrane layer may be of any suitable microporous material for lateral flow membranes. Such materials are known in the art and include nitrocellulose, nylon, cellulose acetate, glass fibres, cross-linked dextran and other porous polymers. Preferably, the membrane layer is a nitrocellulose layer. Nitrocellulose membrane layers for lateral flow assays are well-known in the art and are composed of interconnected nitrocellulose fibres.

The support layer is a liquid-impermeable support layer. Such support layers are well-known in the art and are often referred to as 'backing'. Suitable support layers include polymeric materials such as for example polyester, polypropylene, polyethylene, acrylic (co)polymers, vinylacrylic polymers and heteropolysaccharides.

The membrane assembly according to the invention is preferably applied in a lateral flow immunoassay. In lateral flow immunoassays, a receptor that is able to bind the ligand or analyte to be detected in the test fluid is immobilised in the microporous membrane layer through which the test fluid flows. Therefore, the membrane assembly preferably comprises a ligand-binding molecule immobilised in the membrane layer. Ligand-binding molecules or receptors are known in the art and include for example antibodies and aptamers.

Typically in lateral flow immunoassays, such receptor or ligand-binding molecule is immobilised in the form of one or more test lines or spots and often a control line or spot that are applied on the membrane layer by spraying techniques such as inkjet printing or other aerosol spraying techniques. The membrane layer then comprises one or more detection zones wherein ligand-binding molecules are immobilised. Each detection zone may comprise one or more lines or spots with immobilised ligand-binding molecule. Preferably therefore in the membrane assembly according to the invention, the ligand-binding molecule is immobilised in the membrane layer in one or more detection zones and the membrane layer has, at least in the one or more detection zones, the first thickness. The membrane layer may have the first thickness over its entire length, but preferably the membrane layer has the first thickness in the detection zone(s) and the second thickness as hereinbefore defined outside the one or more detection zones, i.e. upstream, downstream and between the one or more detection zones.

In order to visualise the receptor-ligand pairs formed in the detection zones, markers such as for example immunolabels are typically used. Very often, the liquid to be analysed first flows through a so-called conjugate pad before it flows through the lateral flow membrane. The conjugate pad comprises a moveable conjugate of marker and ligand-binding molecule. If the liquid to be analysed flows through the conjugate pad, the conjugate binds to the ligand and the ligand/conjugate combination flows with the liquid through the membrane layer. In the membrane layer the ligand-conjugate combination binds to immobilised ligand-binding molecules. Alternative to a conjugate pad upstream of the lateral flow membrane, the lateral flow membrane may comprises one or more conjugate zones that comprise a moveable conjugate of marker and ligand-binding molecule. Such conjugate zones are preferably located upstream of a detection zone. If the membrane layer comprises one or more conjugate zones, it is preferred that the membrane layer has the first thickness in the one or more conjugate zones. More preferably, the membrane layer has the first thickness in the one or more detection zones and in the one or more conjugate zones and has the second thickness outside these zones. The membrane layer may have the first thickness in the one or more detection zones and the second thickness outside these zones, including any conjugate zones.

In order to allow for improved binding of ligand to ligand-binding molecule in the detection zones and, if present, in the conjugate zones, it is preferred to have a relatively low flow velocity of liquid to be analysed in the detection and conjugate zones. Therefore, the width of the membrane assembly in detection and/or conjugate zones wherein the membrane layer has the first thickness, i.e. a thickness in the range of from 20 to 80 µm, is preferably larger than the width of the assembly in zones wherein the membrane layer has the larger, second thickness. More preferably, the width of the assembly in detection and/or conjugate zones with the first thickness is such that the flow velocity in such zones is equal or lower, preferably lower, than the flow velocity outside such zones. Preferably, the flow velocity in such zone is in the range of from 25 to 100%, more preferably of from 50 to 95% of the flow velocity outside such zones. Thus, the product of width of the assembly and thickness of the membrane layer in zones with the first thickness is preferably not smaller than such product in zones with the second thickness.

The invention further relates to a lateral flow immunoassay device comprising the membrane assembly according to the invention. Lateral flow immunoassay devices are well-known in the art and are for example described in US2006/0205059, US 5,252,496 and US 5,591,645. The membrane assembly may be used in any suitable lateral flow immunoassay device known in the art.

Typically, lateral flow immunoassays devices comprise a reaction zone comprising a lateral flow membrane with immobilised ligand-binding molecule supported on a liquid-impermeable support layer, a sample addition zone upstream of the reaction zone and an absorbing zone downstream of the reaction zone. Reference herein to upstream or downstream is with respect to the direction of the lateral fluid flow. Test fluid is added to the sample addition zone that typically comprises a filter pad in which the liquid is absorbed. By the action of capillary forces, the liquid flows from the sample addition zone through the reaction zone to the absorbing zone. The device may comprise a so-called conjugate zone comprising moveable immunolabels or other markers that bind to the ligand to be detected in the test liquid, between the sample addition zone and the reaction zone, so that test liquid is forced to flow through the conjugate zone. The markers will then bind to the ligands in the test fluid and labeled ligands flow through the test zone and are bound to immobilised ligand-binding molecules in the reaction zone where they can be visualised. Alternatively, the membrane layer comprises one or more conjugate zones as described hereinabove.

The lateral flow immunoassay device according to the invention preferably is a lateral flow immunoassay device comprising a microporous membrane layer comprising ligand-binding molecule that is applied to the membrane layer by means of inkjet printing or another spraying technique.

The membrane assembly according to the invention is preferably manufactured by first providing a liquid-impermeable support layer and then applying a solution of the material of which the membrane layer is composed in a suitable solvent on the support layer. The solvent is then evaporated and a membrane assembly of a membrane layer supported on the support layer is obtained. A membrane layer having an alternating first and second thickness over its entire width is obtained by providing a liquid-impermeable support layer having elongate protrusions with a height that is similar to the difference in thickness between the first and second thickness in the membrane layer, i.e. preferably in the range of from 20 to 180 µm, preferably of from 80 to 120 µm, to be obtained. In fact, the support layer acts as a mould for the membrane layer to be obtained. By applying the membrane material as a liquid solution, a membrane assembly, i.e. membrane layer plus support layer, of a constant height is obtained. Elongate membrane assemblies according to the invention suitable to be used in lateral flow immunoassay devices, can be cut from the assembly of support and membrane layer thus obtained.

The invention is further illustrated by means of the following, non-limiting drawings.

### Detailed Description of the Drawings

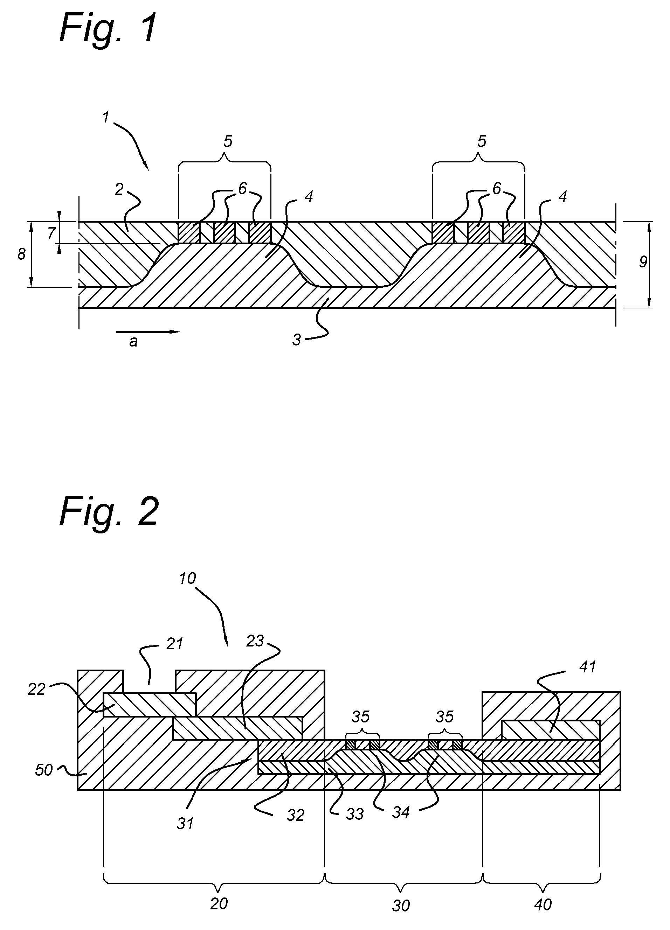

In Figure 1 is schematically shown a longitudinal section of membrane assembly 1 according to the invention comprising microporous membrane layer 2 and support layer 3. Support layer 3 has protrusions 4 over its entire width. Membrane layer 2 comprises detection zones 5 comprising antibodies immobilised in test lines 6. Each detection zone 5 comprises three test lines 6. Membrane layer 2 has a first thickness 7 in detection zones 5 and a second thickness 8 outside the detection zones. Upstream of each detection zone 5, the thickness of membrane layer 2 is gradually decreasing from second thickness 8 to first thickness 7. Membrane assembly 1 has a height 9 that is constant over the entire length of membrane assembly 1. Reference herein to upstream is with reference to the lateral flow direction. Arrow 'a' indicates the flow direction.

In Figure 2 is shown a longitudinal section of lateral flow immunoassay device 10 according to the invention. Device 10 comprises sample addition zone 20, reaction zone 30 and absorption zone 40 in a housing 50. During normal operation of the device, test liquid is applied via application window 21 on sample pad 22. Through the action of capillary forces, test liquid flows from sample path 22 via conjugate pad 23 to membrane assembly 31 in reaction zone 30. Conjugate path 23 comprises moveable immunolabels that bind to the ligand to be detected in the test liquid and flow with the test liquid to reaction zone 30. Membrane assembly 31 comprises membrane layer 32 and support layer 33. Support layer has protrusions 34 so that membrane layer 32 has a smaller thickness in detection zones 35. Absorption zone 40 comprises absorbent pad 41.

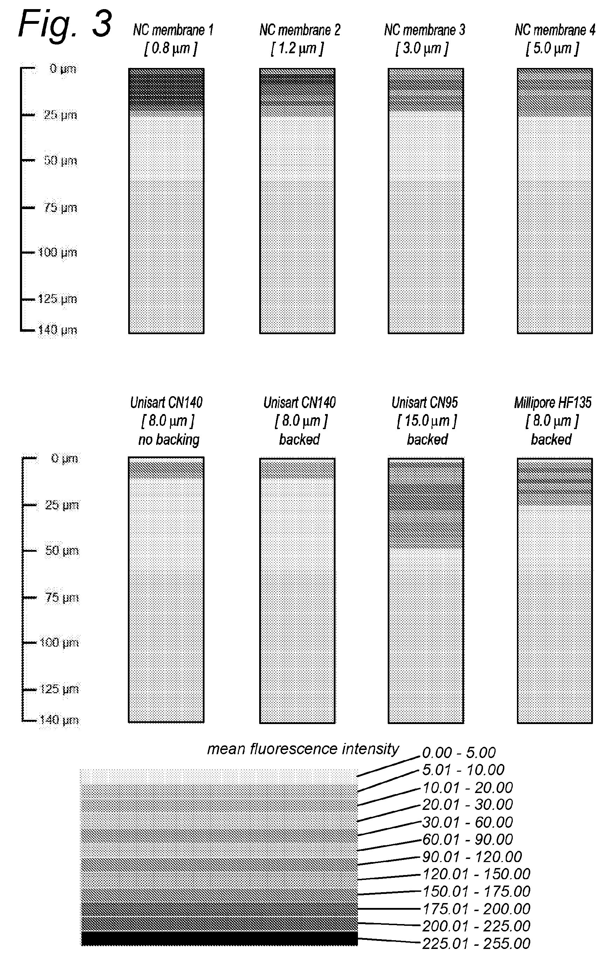

In Figure 3 is shown the results of confocal laser scanning microscopy experiments showing the depth of antibody immobilisation in different nitrocellulose membrane layers as described in further detail in the examples below.

### Examples

The following examples show that ligand-binding molecules that are immobilised in a microporous lateral flow membrane by means of a spraying technique such as for example inkjet printing, are mainly immobilised in the upper part of the membrane.

AlexaFluor647 labeled IgG molecules (Molecular Probes A21235; 200 µg/mL) were sprayed in a line-format on:
- four experimental nitrocellulose microporous membranes having a nominal pore size of 0.8 µm (NC membrane 1), 1.2 µm (NC membrane 2), 3.0 µm (NC membrane 3) and 5.0 µm (NC membrane 4);
- three commercial membranes, each supported by a backing layer (Sartorius Unisart CN95, Sartorius Unisart CN140 and Millipore HF135 with nominal pore sizes of 15.0, 8.0 and 8.0 µm, respectively; and
- an Unisart CN140 membrane without a backing layer (nominal pore size 8.0 µm).
The membranes each have a width of 0.5 cm and lines of IgG-AlexaFluor647 were sprayed by means of a CAMAG Linomat IV TLC sprayer with an amount of 1 µL per 0.5 cm, i.e. 200 ng per line and per strip. Following drying of the membranes (overnight at 37°C) the position of the immobilised fluorescent antibody molecules as measured from the top of the membrane was assessed by Confocal Laser Scanning Microscopy (CLSM) in the Z-stacking mode. Hereto, the fluorescence of membrane slices having a height of 3 µm was detected, starting from the top of the membrane. The fluorescence of a total of 21 slices in each of the membranes tested is shown in Figure 3.

In Figure 3, the relative intensity of the fluorescence is shown. In general, the smaller the nominal pore size, the smaller the depth of the zone where antibody molecules had been immobilised. Antibody molecules were immobilised in a depth of 12 µm (Unisart CN140) to 51 µm (Unisart CN95) from the top of the membranes. In the Table below is given for each membrane the depth of the membrane layer at which the relative fluorescence intensity was less than 5 Relative Fluorescence Units.

**Table - Depth at which the fluorescence is below 5 Relative Fluorescence Units**

| Membrane | Depth (µm) |
|---|---|
| NC membrane 1 | 27 |
| NC membrane 2 | 27 |
| NC membrane 3 | 24 |
| NC membrane 4 | 27 |
| Unisart CN140, no backing | 12 |
| Unisart CN140, backed | 12 |
| Unisart CN95, backed | 51 |
| Millipore HF135, backed | 27 |

## Claims

1. An elongate membrane assembly having a length, a width and a height, the assembly comprising a microporous membrane layer supported on a liquid-impermeable support layer and the assembly being suitable for lateral flow of a liquid through the membrane layer under the action of capillary forces, wherein the assembly has a constant height and at least part of the membrane layer has a first thickness in the range of from 20 to 80 µm over the entire width of the assembly.

2. A membrane assembly according to claim 1, wherein the microporous membrane is a nitrocellulose membrane.

3. A membrane assembly according to claim 1 or 2, wherein the first thickness is in the range of from 20 to 60 µm.

4. A membrane assembly according to any one of the preceding claims, wherein the entire membrane layer has the first thickness.

5. A membrane assembly according to any one of claims 1 to 3, wherein the membrane layer has in lateral direction alternating the first thickness and a second thickness of from at least 100 µm over the entire width of the assembly.

6. A membrane assembly according to claim 5, wherein the second thickness is at most 200 µm.

7. A membrane assembly according to claim 5 or 6, wherein the thickness of the membrane layer is gradually decreasing in lateral direction from the second to the first thickness.

8. A membrane assembly according to any one of the preceding claims further comprising a ligand-binding molecule immobilised in the membrane layer.

9. A membrane assembly according to claim 8, wherein the ligand-binding molecule is immobilised in the membrane layer in one or more detection zones and wherein the membrane layer has at least in the one or more detection zones the first thickness.

10. A membrane assembly according to claim 9, wherein the membrane layer further comprises one or more conjugate zones comprising a moveable conjugate of a ligand-binding molecule and a marker, wherein the membrane layer has the first thickness in the one or more conjugate zones.

11. A membrane assembly according to 9 or 10 and any one of claims 5 to 7, wherein the membrane layer has the second thickness outside the one or more detection zones and the one of more conjugate zones.

12. A membrane assembly according to claim 11, wherein the width of the membrane assembly is larger in the one or more detection zones and in the one or more conjugate zones than outside the detection and conjugate zones.

13. A membrane assembly according to claim 12, wherein the product of width of the assembly and thickness of the membrane layer in any one of the detection and conjugate zones is not smaller than the product of width of the assembly and thickness of the membrane layer outside the detection and conjugate zones.

14. A lateral flow immunoassay device comprising the membrane assembly according to any one of the preceding claims.

## Patentansprüche

1. Längliche Membrananordnung mit einer Länge, einer Breite und einer Höhe, wobei die Anordnung eine auf einer flüssigkeitsundurchlässigen Trägerschicht getragene mikroporöse Membranschicht umfasst und die Anordnung für den seitlichen Fluss einer Flüssigkeit durch die Membranschicht unter der Wirkung von Kapillarkräften geeignet ist, wobei die Anordnung eine konstante Höhe aufweist und zumindest ein Teil der Membranschicht eine erste Dicke in dem Bereich von 20 bis 80 µm über die gesamte Breite der Anordnung aufweist.

2. Membrananordnung nach Anspruch 1, wobei die mikroporöse Membran eine Nitrocellulose-Membran ist.

3. Membrananordnung nach Anspruch 1 oder 2, wobei die erste Dicke im Bereich von 20 bis 60 µm liegt.

4. Membrananordnung nach einem der vorangehenden Ansprüche, wobei die gesamte Membranschicht die erste Dicke aufweist.

5. Membrananordnung nach einem der Ansprüche 1 bis 3, wobei die Membranschicht in seitlicher Richtung abwechselnd die erste Dicke und eine zweite Dicke von mindestens 100 µm über die gesamte Breite der Anordnung aufweist.

6. Membrananordnung nach Anspruch 5, wobei die zweite Dicke höchstens 200 µm beträgt.

7. Membrananordnung nach Anspruch 5 oder 6, wobei die Dicke der Membranschicht in seitlicher Richtung von der zweiten zu der ersten Dicke allmählich abnimmt.

8. Membrananordnung nach einem der vorangehenden Ansprüche, weiterhin umfassend ein in der Membranschicht immobilisiertes ligandenbindendes Molekül.

9. Membrananordnung nach Anspruch 8, wobei das ligandenbindende Molekül in der Membranschicht in einer oder mehreren Nachweiszonen immobilisiert ist und wobei die Membranschicht zumindest in der einen oder den mehreren Nachweiszonen die erste Dicke aufweist.

10. Membrananordnung nach Anspruch 9, wobei die Membranschicht weiterhin ein oder mehrere Konjugatzonen mit einem beweglichen Konjugat aus einem ligandenbindenden Molekül und einem Marker umfasst, wobei die Membranschicht die erste Dicke in der einen oder den mehreren Konjugatzonen aufweist.

11. Membrananordnung nach Anspruch 9 oder 10 und einem der Ansprüche 5 bis 7, wobei die Membranschicht die zweite Dicke außerhalb der einen oder mehreren Nachweiszonen und der einen oder mehreren Konjugatzonen aufweist.

12. Membrananordnung nach Anspruch 11, wobei die Breite der Membrananordnung in der einen oder den mehreren Nachweiszonen und in der einen oder den mehreren Konjugatzonen größer ist als außerhalb der Nachweis- und Konjugatzonen.

13. Membrananordnung nach Anspruch 12, wobei das Produkt aus der Breite der Anordnung und der Dicke der Membranschicht in jeder der Nachweis- und Konjugatzonen nicht kleiner ist als das Produkt aus der Breite der Anordnung und der Dicke der Membranschicht außerhalb der Nachweis- und Konjugatzonen.

14. Seitliche Flussimmunoassay-Vorrichtung mit der Membrananordnung nach einem der vorangehenden Ansprüche.

## Revendications

1. Ensemble à membrane allongé ayant une longueur, une largeur et une hauteur, l'ensemble comprenant une couche de membrane microporeuse supportée par une couche de support imperméable aux liquides et l'ensemble étant adapté à l'écoulement latéral d'un liquide à travers la couche de membrane sous l'action de forces capillaires, l'ensemble ayant une hauteur constante et au moins une partie de la couche de membrane ayant une première épaisseur dans la gamme allant de 20 à 80 µm sur toute la largeur de l'ensemble.

2. Ensemble à membrane selon la revendication 1, dans lequel la membrane microporeuse est une membrane de nitrocellulose.

3. Ensemble à membrane selon la revendication 1 ou 2, dans lequel la première épaisseur est dans la gamme allant de 20 à 60 µm.

4. Ensemble à membrane selon l'une quelconque des revendications précédentes, dans lequel toute la couche de membrane a la première épaisseur.

5. Ensemble à membrane selon l'une quelconque des revendications 1 à 3, dans lequel la couche de membrane présente une alternance dans la direction latérale de la première épaisseur et d'une seconde épaisseur d'au moins 100 µm sur toute la largeur de l'ensemble.

6. Ensemble à membrane selon la revendication 5, dans lequel la seconde épaisseur est de 200 µm maximum.

7. Ensemble à membrane selon la revendication 5 ou 6, dans lequel l'épaisseur de la couche de membrane diminue progressivement dans la direction latérale de la seconde à la première épaisseur.

8. Ensemble à membrane selon l'une quelconque des revendications précédentes, comprenant en outre une molécule de liaison à un ligand immobilisée dans la couche de membrane.

9. Ensemble à membrane selon la revendication 8, dans lequel the molécule de liaison à un ligand est immobilisée dans la couche de membrane dans une ou plusieurs zones de détection, et dans lequel la couche de membrane a la première épaisseur au moins dans une ou plusieurs zones de détection.

10. Ensemble à membrane selon la revendication 9, dans lequel la couche de membrane comprend en outre une ou plusieurs zones de conjugué comprenant un conjugué mobile d'une molécule de liaison à un ligand et un marqueur, la couche de membrane ayant la première épaisseur dans une ou plusieurs zones de conjugué.

11. Ensemble à membrane selon 9 ou 10 et l'une quelconque des revendications 5 à 7, dans lequel la couche de membrane a la seconde épaisseur à l'extérieur des une ou plusieurs zones de détection et de l'une ou plusieurs zones de conjugué.

12. Ensemble à membrane selon la revendication 11, dans lequel la largeur de l'ensemble à membrane est plus grande dans les une ou plusieurs zones de détection et dans les une ou plusieurs zones de conjugué qu'à l'extérieur des zones de détection et de conjugué.

13. Ensemble à membrane selon la revendication 12, dans lequel le produit de la largeur de l'ensemble et de l'épaisseur de la couche de membrane dans l'une quelconque des zones de détection et de conjugué n'est pas inférieur au produit de la largeur de l'ensemble et de l'épaisseur de la couche de membrane à l'extérieur des zones de détection et de conjugué.

14. Dispositif d'immunoessai à écoulement latéral comprenant l'ensemble à membrane selon l'une quelconque des revendications précédentes.
